# EUROPEAN PATENT APPLICATION

(11) **EP 1 612 205 A1**
(43) Date of publication of application: **04.01.2006**
(21) Application number: 04724844.8
(22) Date of filing: 31.03.2004
(51) Int. Cl.: C07C 315/04, C07C 317/22, B41M 5/30

(54) **METHOD FOR PRODUCING 3,3';-DIALLYL-4,4';-DIHYDROXYDIPHENYLSULFONE**

(30) Priority: 03.04.2003 JP 2003100352; 29.08.2003 JP 2003306348
(71) Applicant: Sanko Chemical Industry Co., Ltd., Tokyo 103-0023 (JP); Yanagida, Shozo, Hyogo 666-0133 (JP)
(72) Inventor: YANAGIDA, Shozo, Kawanishi-shi,2Hyogo 666-0133 (JP); ENOKIDA, Hirotaka, Kouza-gun, Kanagawa 2530101 (JP); FUJIMOTO, Masaki, Kawaguchi-shi, Saitama 3330811 (JP); NAKAMURA, Katsunori, Fujimi-shi, Saitama 3540031 (JP); YAMAMOTO, Tetsushi, Minoo-shi, Osaka 5620031 (JP); WADA, Yuji, Toyonaka-shi, Osaka 5600005 (JP)
(74) Representative: Gille Hrabal Struck Neidlein Prop Roos
(86) International application number: PCT/JP2004/004719
(87) International publication number: WO 2004/089883

(57) **Abstract**

The present invention is **characterized by** subjecting 4,4'-diallyloxydiphenyl sulfone to a rearrangement reaction under microwave irradiation, preferably in molten state, to produce objective 3,3'-diallyl-4,4'-dihydroxydiphenyl sulfone efficiently in a short time, in high yield and in high purity. Further preferably, by carrying out said reaction in substantially oxygen-free atmosphere in the presence of at least one compound selected from a group consisting of an antioxidant, an organic basic compound and a chelate compound, the above-described object can be attained in more preferable manner.

## Description

### Technical Field

The present invention relates to a production method for 3,3'-diallyl-4,4'-dihydroxydiphenyl sulfone, a useful substance as a developer of thermal recording material or a polymer additive.

### Background of the Invention

3,3'-Diallyl-4,4'-dihydroxydiphenyl sulfone is a useful substance as a developer for a thermal recording material or a polymer additive. It is proposed to synthesize 3,3'-diallyl-4,4'-dihydroxydiphenyl sulfone by Claisen rearrangement of 4,4'-diallyloxydiphenyl sulfone. For example, 4,4'-diallyloxydiphenyl sulfone is reacted in a trichlorobenzene solvent at 216 to 219ºC for 10 hours to obtain the substance with melting point of 139 to 144ºC in 93.3% yield (patent literature 1). Also, 4,4'-diallyloxydiphenyl sulfone, having alkali content reduced to not higher than 50 ppm as based on NaOH is reacted in a paraffinic solvent at 205 to 210ºC for 7 hours to obtain 96.2% (JP-A-2002-3006) or 97.1% (JP-A-2002-30065) as composition ratio (purity)determined by HPLC after purification. Any of these methods had defect in that the reaction should be carried out in long period, for example, 7 to 10 hours and at high temperature of not lower than 200ºC. Low yield such as about 70% as mole ratio based on charged raw material was also a problem.

Therefore, it has been desired to develop a more efficient (shorter reaction time and higher yield) production method for high purity 3,3'-diallyl-4,4'-dihydroxydiphenyl sulfone with small byproducts mixed in.

### Summary of the Invention

The present inventors have extensively studied a way to solve the above-described problems and found that, by rearrangement reaction of 4,4'-diallyloxydiphenyl sulfone under microwave irradiation, 3,3'-diallyl-4,4'-dihydroxydiphenyl sulfone can be produced in a short time of about several minutes to 30 minutes in high yield, on one hand conventional method took 7 hours to 10 hours.

The present inventors have also extensively studied a way to suppress side reactions and also found that by subjecting 4,4'-diallyloxydiphenyl sulfone to a rearrangement reaction, in particular a rearrangement reaction under microwave irradiation, preferably in substantially oxygen-free atmosphere in the presence of at least one compound selected from a group consisting of an antioxidant, an organic basic compound and a chelate compound, preferably in the presence of at least one compound of an organic basic compound or/and a chelate compound, reaction time can significantly be reduced and a target 3,3'-diallyl-4,4'-dihydroxydiphenyl sulfone, can be produced in high yield with low byproducts.

That is, the present invention relates to:
1. A production method for 3,3'-diallyl-4,4'-dihydroxydiphenyl sulfone characterized by subjecting 4,4'-diallyloxydiphenyl sulfone to a rearrangement reaction under microwave irradiation.
2. The production method for 3,3'-diallyl-4,4'-dihydroxydiphenyl sulfone according to claim 1, characterized in that the rearrangement reaction is carried out in molten state.
3. The production method for 3,3'-diallyl-4,4'-dihydroxydiphenyl sulfone according to claim 2, characterized in that the rearrangement reaction is carried out at 230 to 300ºC.
4. The production method for 3,3'-diallyl-4,4'-dihydroxydiphenyl sulfone according to claim 1 or claim 2, characterized in that the rearrangement reaction is carried out in substantially oxygen-free atmosphere.
5. The production method for 3,3'-diallyl-4,4'-dihydroxydiphenyl sulfone according to claim 1, characterized in that the rearrangement reaction is carried out in the presence of a basic substance.
6. The production method for 3,3'-diallyl-4,4'-dihydroxydiphenyl sulfone according to claim 1, characterized in that the rearrangement reaction is carried out in the presence of an antioxidant.
7. The production method for 3,3'-diallyl-4,4'-dihydroxydiphenyl sulfone according to claim 6, characterized in that the rearrangement reaction is carried out in the presence of ascorbic acid as an antioxidant.
8. The production method for 3,3'-diallyl-4,4'-dihydroxydiphenyl sulfone according to claim 1, characterized in that the rearrangement reaction is carried out in the presence of a chelate agent.
9. The production method for 3,3'-diallyl-4,4'-dihydroxydiphenyl sulfone according to claim 8, characterized in that said chelate agent is ethylenediamine tetraacetic acid or a chelate agent comprising a fused ring containing a nitrogen-containing aromatic ring.
10. The production method for 3,3'-diallyl-4,4'-dihydroxydiphenyl sulfone according to claim 9, characterized in that the chelate agent comprising a fused ring is phenanthroline.
11. A production method for 3,3'-diallyl-4,4'-dihydroxydiphenyl sulfone characterized by subjecting 4,4'-diallyloxydiphenyl sulfone to a rearrangement reaction under microwave irradiation at 230 to 300ºC in the presence of a basic substance or a chelate agent.

### Best Mode to Carry Out the Invention

The present invention is carried out by subjecting 4,4'-diallyloxydiphenyl sulfone to a rearrangement reaction under microwave irradiation.

A raw material, 4,4'-diallyloxydiphenyl sulfone, of the present invention can be produced by subjecting 4,4'-dihydroxydiphenyl sulfone to a reaction with allyl chloride or allyl bromide in an organic solvent in the presence of an alkaline substance such as an alkaline metal hydroxide and an alkaline earth metal hydroxide (JP-A-60-169456). Alkali components contained in a raw material, 4,4'-diallyloxydiphenyl sulfone, may promote formation of byproducts such as 3,3'-diallyl-4,2'-dihydroxydiphenyl sulfone or 5-(3-allyl-4-hydroxy-phenylsulfonyl)-1-oxa-2-methylindane in a rearrangement reaction of the present invention. These byproducts are difficult to be removed, which may thus impair quality such as background decline when used as a developer of thermal recording material, therefore it is preferable to use 4,4'-diallyloxydiphenyl sulfone whose alkali content is not higher than 100 ppm.

In the present invention, to suppress side reactions or a polymerization reaction, it is preferable to carry out the reaction in inert gas atmosphere such as nitrogen or argon in substantially oxygen-free state.

As a combination of a chelate agent and an antioxidant, a combination of 1,10-phenanthrolines or EDTA and ascorbic acid is preferable.

Microwave used in the present invention may be any one of electromagnetic wave having frequency of usually at 300 MHz to 30 GHz. An industrially used microwave generator adopts at 2450 MHz or 918 MHz, therefore such the generator may usually be used. Irradiation time depends on charge amount, watt of a microwave irradiator and so on and definite time is not specified unconditionally, however, it is usually 1 to 60 minutes at 100W to 10 KW after elevation to reaction temperature. In view of easiness of reaction control, it is preferable to complete a reaction in about 5 to 30 minutes. Reaction temperature is controlled in the range from 150 to 350ºC, preferably 230 to 300ºC and more preferably 240 to 290ºC by intermittent irradiation (on-off) of electromagnetic wave. Experimental equipment with microwave irradiation is manufactured and sold from, for example, Milestone Co., CEM Co., Microelectronics Co., and the like.

A rearrangement reaction in the present invention can be carried out in any of non-solvent or the presence of a solvent. When a solvent is used, an inert solvent with high boiling point is preferable. For example, a polar solvent such as N,N-dimethylformamide, N,N-dimethylacetamide, N-methyl-2-pyrrolidinone and dimethylsulfoxide; chlorobenzenes such as o-dichlorobenzene and trichlorobenzene; and aliphatic hydrocarbons with high boiling point can be used. When a solvent is used, a raw material, 4,4'-diallyloxydiphenyl sulfone, is usually used by dissolving or dispersing into the solvent. In this case, reaction temperature is usually from about 190 to 220ºC. A small amount of a solvent may be used to the extent of moistening a raw material, for example, one part per several parts of 4,4'- diallyloxydiphenyl sulfone, specifically, about not higher than 1/3, preferably not higher than 1/4 and more preferably not higher than 1/5.

When a solvent is not used or only a small amount of a solvent is used to the extent of moistening a raw material, it is preferable that the raw material is molten in advance, and subjected to a rearrangement reaction under microwave irradiation in this state. In this way, reaction time can be further reduced while suppressing byproducts to a low level. To melt a raw material, microwave may be irradiated to a raw material or other methods may be adopted such as heating with a conventional electric heater or a heater using heating media.

In the present invention, in view of good charging efficiency into equipment and elimination of solvent recovery, it is preferable to carry out the rearrangement reaction without a solvent.

In the present invention, to suppress side reactions or a polymerization reaction, it is preferable to carry out the reaction in inert gas atmosphere such as nitrogen or argon in substantially oxygen-free state. It is also preferable, to suppress side reactions, that the reaction is carried out in the presence of at least one compound selected from a group consisting of an antioxidant, an organic basic compound and a chelate compound. It is also a preferable embodiment that the reaction is carried out in the co-presence of (1) an antioxidant and (2) at least one compound of an organic basic compound or/and a chelate compound.

Examples of an antioxidant include ascorbic acid, tocopherols, methoxyhydroquinone and butylhydroquinone and ascorbic acid is more preferable. Salts thereof may also be used as long as they do not lose the effect of the present invention.

The amount of the addition of an antioxidant of not higher than 1% by mass (hereinafter, % by mass unless otherwise specified) based on 4,4'-diallyloxydiphenyl sulfone is enough and it is usually about 0.01% by weight to 0.5% by weight.

An example of an organic basic compound includes N,N-di-lower-alkyl aromatic amines such as N,N-dimethylaniline, N,N-diethylaniline and dimethylaminopyridine; and nitrogen-containing cyclic compounds such as hexamethylenetetramine, quinuclidine, quinoline, isoquinoline, quinaldine and quinoxaline. Here, a lower alkyl group is preferably an alkyl group with carbon atoms of 1 to 6, preferably 1 to 3. An aromatic ring in aromatic amines includes a 5- to 6-membered, preferably a 6-membered aromatic ring. As a nitrogen-containing cyclic compound, such one having carbon atoms of about 5 to 15, preferably about 6 to 12 is preferable (nitrogen atoms are preferably 1 to 4 and preferably not more than half of total atoms forming a ring). Among these, N,N-di-lower alkyl aromatic amines are preferable.

As a chelate agent used in the present invention, nitrogen-containing heterocyclic chelate agents or aliphatic polyamino polyacetic acids are included. As nitrogen-containing heterocycles are included, for example, a pyridine ring or a fused pyridine ring. Nitrogen atom-containing heterocyclic chelate agents are preferably aromatic heterocyclic compounds with, for example, 1 to 3, preferably 2 said pyridine rings or fused pyridine rings and carbon atoms of 8 to 18, preferably 9 to 12, wherein said compounds may be substituted with a hydroxyl group, a phenyl group, and the like, and are included, for example, 1,10-phenanthrolines such as 1,10-phenanthroline, 4,7-diphenyl-1,10-phenanthroline, 2,9-dimethyl-1,10-phenanthroline; 8-quinolinol(oxine), 2,2'-biquinoline, 2,2'-bipyridyl, 2,2',2''-terpyridine, 1,8-naphthyridines, etc. An aliphatic polyamino polyacetic acid includes an aliphatic hydrocarbon compound with carbon atoms of 2 to 5, preferably2 to 3 substituted with 2 to3 amino groups, preferably 2 amino groups and all hydrogen atoms in said amino groups are substituted with acetic acid, for example, ethylendiamine tetraacetic acid (EDTA), propylendiamine tetraacetic acid (PDT), etc. A preferable nitrogen atom containing hetero cyclic chelate agent includes 1,10-phenanthrolines, and 1,10-phenanthroline is most preferable. As an aliphatic polyamino polyacetic acid, EDTA is preferable. The amount of the addition of a chelate agent of not higher than 1% by mass (hereinafter, % by mass unless specified otherwise) based on 4,4'-diallyloxydiphenyl sulfone is enough and it is usually about 0.01% to 0.5% by mass.

As a combination of a chelate agent and an antioxidant, a combination of a chelate agent, preferably 1,10-phenanthrolines or EDTA and ascorbic acid as an antioxidant is preferable.

The amount of the addition of a side reaction suppressing agent of not higher than 1% by mass (hereinafter, % by mass is used unless otherwise specified) based on 4,4'-diallyloxydiphenyl sulfone is enough and it is usually about 0.01% to 0.5% .

A rearrangement reaction may be carried out by any of a batch system where 4,4'-diallyloxydiphenyl sulfone is charged in a reactor all at once, followed by microwave irradiation; a semi-batch system where 4,4'-diallyloxydiphenyl sulfone is supplied continuously or in portion wise into a reactor under microwave irradiation; or a flow-reaction system where 4,4'-diallyloxydiphenyl sulfone is fed continuously into a reactor under microwave irradiation and a product is continuously discharged.

The reaction products can be confirmed by high performance liquid chromatography (HPLC) analysis. After the end of a reaction, content of 3,3'-diallyl-4,4'-dihydroxydiphenyl sulfone in reaction products is not lower than 85% and preferably not lower than 90%. This substance can be subjected to a conventional purification method, such as dissolving in an aqueous alkaline solution, followed by acidic deposition; or dissolving in an organic solvent while heating, followed by cooling to deposit crystal and isolation by filtering; or combination of both methods (acidic deposition and recrystallization from an organic solvent). Purity of purified 3,3'-diallyl- 4,4'-dihydroxydiphenyl sulfone thus obtained is usually not lower than 97% (area % by high performance liquid chromatography; the same hereinafter) and yield thereof is not lower than 80% (based on charged amount; the same hereinafter).

### Examples

The present invention will be explained in more detail using Examples, however, the present invention should not be limited thereto.

### Example 1

To a quartz flask equipped with a temperature sensor and a magnetic stirrer, 10.00 g of 4,4'-diallyloxydiphenyl sulfone and 0.01 g of N,N-dimethylaniline were charged, followed by purging with nitrogen. Under nitrogen flow, 2450 MHz microwave was irradiated at 100 W, followed by melting at 160ºC and then reaction was continued for 5 minutes while maintaining reaction temperature at 280ºC by on-off control of irradiation. The reaction products were analyzed by high performance liquid chromatography (area % by high performance liquid chromatography; the same hereinafter): 3,3'-diallyl-4,4'-dihydroxydiphenyl sulfone (hereinafter abbreviated as the di-rearranged compound) 89.5%, 3-allyl-4-hydroxy-4'-allyloxydiphenyl sulfone (hereinafter abbreviated as the mono-rearranged compound) 1.4%, 5-(3-allyl-4-hydroxyphenylsulfonyl)- 1-oxa-2-methylindane (hereinafter abbreviated as the indane-type compound) 1.1%, 3-allyl-4,4'-dihydroxy- diphenyl sulfone (hereinafter abbreviated as the mono-allyl compound) 1.5%, isomers 0.8% and dimers 1.8%.

These reaction products are dissolved in an aqueous solution of 10% by weight of sodium hydroxide, and then a small amount of activated carbon is added thereto , followed by heating and stirring for decolorization treatment. After separation of activated carbon by filtration, hydrochloric acid is added to neutralize and precipitate crystals. Thereby, purified 3,3'-diallyl-4,4'-dihydroxydiphenyl sulfone with purity of 96% to 98% can be obtained in 80% to 90% yield (% by mass; the same hereinafter).

### Example 2

To a quartz flask equipped with a temperature sensor and a magnetic stirrer, 10.00 g of 4,4'-diallyloxydiphenyl sulfone and 0.01 g of N,N-dimethylaniline were charged, followed by purging with nitrogen. Under nitrogen flow, 2450 MHz microwave was irradiated at 100 W, followed by melting at 160ºC and reaction was continued for 20 minutes while maintaining reaction temperature at 255ºC by on-off control of irradiation. Thus obtained-reaction products were analyzed by high performance liquid chromatography: the di-rearranged compound 91.1%, the mono-rearranged compound 1.6%, the indane-type compound 1.0%, the mono-allyl compound 1.1%, isomers 0.6% and dimers 1.4%.

By purifying thus obtained reaction product by the method according to Example 1, purified 3,3'-diallyl-4,4'-dihydroxydiphenyl sulfone with purity of 96% to 98% can be obtained in 80% to 90% yield.

### Example 3

To a quartz flask equipped with a temperature sensor and a magnetic stirrer, 10.00 g of 4,4'-diallyloxydiphenyl sulfone was charged, followed by purging with nitrogen. Under nitrogen flow, 2450 MHz microwave was irradiated at 100 W, followed by melting at 160ºC and reaction was continued for 5 minutes while maintaining reaction temperature at 280ºC by on-off control of irradiation. Thus obtained reaction products were analyzed by high performance liquid chromatography: the di-rearranged compound 87.4%, the mono-rearranged compound 1.9%, the indane-type compound 1.7%, the mono-allyl compound 2.6%, isomers 1.7% and dimers 1.9%. By purifying thus obtained reaction product by the method according to Example 1, purified 3,3'-diallyl-4,4'-dihydroxydiphenyl sulfone with purity of 96% to 98% can be obtained in 80% to 90% yield.

### Example 4

To a quartz flask equipped with a temperature sensor and a magnetic stirrer, 10.00 g of 4,4'-diallyloxydiphenyl sulfone and 10 mg of 1,10-phenanthroline were charged, followed by purging with nitrogen. Under nitrogen flow, 2450 MHz microwave was irradiated to them at 100 W and, after melting thereof at 160ºC, reaction temperature was elevated to 280ºC and then reaction was continued for 5 minutes while maintaining reaction temperature at 280ºC by on-off control of irradiation. The reaction products were analyzed by high performance liquid chromatography was: the di-rearranged compound 90.5%, the mono-rearranged compound 1.5%, the indane-type compound 1.5%, the mono-allyl compound 1.5%, unidentified compounds 0.8% and dimers 1.6%.
These reaction products are dissolved in an aqueous solution of 10% by weight of sodium hydroxide, and then a small amount of activated carbon is added thereto , followed by heating and stirring for decolorization treatment. After separation of activated carbon by filtration, sulfuric acid of 20% concentration was added to neutralize and precipitate crystals. Thereby, purified 3,3'-diallyl-4,4'-dihydroxydiphenyl sulfone were obtained in amount of 8.45 g (84.5% yield). The content of di-rearranged compound was 97.0% by high performance liquid chromatography analysis.

### Example 5

To a pyrex flask equipped with a temperature sensor and a magnetic stirrer, 10.00 g of 4,4'-diallyloxydiphenyl sulfone, 5 mg of 1,10-phenanthroline and 5 mg of ascorbic acid were charged , followed by purging with nitrogen. Under nitrogen flow, they were heated by an electric heater. After melting thereof at 160ºC, 2450 MHz microwave at 100 W was irradiated to elevate the temperature to 260ºC and reaction was continued for 16 minutes while maintaining reaction temperature at 280ºC by on-off control of irradiation. Analysis value of reaction products thus obtained by high performance liquid chromatography was : the di-rearranged compound 93.6%, the mono-rearranged compound 1.3%, the indane-type compound 1.2%, the mono-allyl compound 0.3%, unidentified components 0.6% and dimers 1.2%. By purifying thus obtained reaction product by the method according to Example 1, 8.70 g (87% yield) of purified 3,3'-diallyl-4,4'-dihydroxydiphenyl sulfone were obtained. Thus obtained products were analyzed by high performance liquid chromatography: Content of di-rearranged compound; 97.5%.

### Example 7

To a Pyrex flask equipped with a temperature sensor and a magnetic stirrer, 10.00 g of 4,4'-diallyloxydiphenyl sulfone, 5 mg of EDTA and 5 mg of ascorbic acid were charged, followed by purging with nitrogen. Under nitrogen flow, 2450 MHz microwave at 100 W was irradiated to elevate the temperature to 260ºC and reaction was continued for 16 minutes while maintaining reaction temperature at 260ºC by on-off control of irradiation. Analysis value of reaction products thus obtained by high performance liquid chromatography was: the di-rearranged compound 91.8%, the mono-rearranged compound 1.1%, the indane-type compound 1.2%, the mono-allyl compound 0.4%, unidentified components 0.8% and dimers 1.9%. By purifying thus obtained reaction product by a method according to Example 1, 8.50 g (85.0% yield) of purified 3,3'-diallyl-4,4'-dihydroxydiphenyl sulfone were obtained. Thus obtained products were analyzed by high performance liquid chromatography: Content of di-rearranged compound; 97.2%.

### Example 8

To a 100 ml flask equipped with a temperature sensor, a stirrer and a condenser, 20 g of trichlorobenzene, 10.00 g of 4,4'-diallyloxydiphenyl sulfone, 10 mg of o-phenanthroline and 10 mg of ascorbic acid were charged, followed by purging with nitrogen. Under nitrogen flow, while heating by an oil bath, reaction was continued at 210ºC for 7 hours. Thus obtained reaction products were analyzed by high performance liquid chromatography: the di-rearranged compound 95.3%, the mono-rearranged compound 2.2%, the indane-type compound 0.7%, the mono-allyl compound 0.1%, unidentified compounds 0.1% and dimers 0.5%. A trichlorobenzene solution of reaction products was extracted with an aqueous solution of 10% by weight of sodium hydroxide while heating. A small amount of activated carbon to the extract and stirring for decolorization treatment. After separation of activated carbon by filtration, sulfuric acid of 20% concentration was added to the filtrate to neutralize and precipitate crystals. Purified 3,3'-diallyl-4,4'-dihydroxydiphenyl sulfone was obtained in amount of 8.80 g (in 88.0% yield). Thus obtained products were analyzed by high performance liquid chromatography: Content of di-rearranged compound; 97.0%.

### Industrial Applicability

The present invention is economically very advantageous because a rearrangement reaction from 4,4'-diallyloxydiphenyl sulfone to 3,3'-diallyl-4,4'-dihydroxydiphenyl sulfone can be carried out in about several minutes to 30 minutes, while conventional method took 7 hours to 10 hours, and that byproducts can be suppressed and an objective compound can be obtained in high yield and the reaction can be carried out even in a non-solvent system.

## Claims

1. A production method for 3,3'-diallyl-4,4'-dihydroxydiphenyl sulfone **characterized by** subjecting 4,4'-diallyloxydiphenyl sulfone to a rearrangement reaction under microwave irradiation.

2. The production method for 3,3'-diallyl-4,4'-dihydroxydiphenyl sulfone according to claim 1, **characterized in that** the rearrangement reaction is carried out in molten state.

3. The production method for 3,3'-diallyl-4,4'-dihydroxydiphenyl sulfone according to claim 2, **characterized in that** the rearrangement reaction is carried out at 230 to 300ºC.

4. The production method for 3,3'-diallyl-4,4'-dihydroxydiphenyl sulfone according to claim 1 or claim 2, **characterized in that** the rearrangement reaction is carried out in substantially oxygen-free atmosphere.

5. The production method for 3,3'-diallyl-4,4'-dihydroxydiphenyl sulfone according to claim 1, **characterized in that** the rearrangement reaction is carried out in the presence of a basic substance.

6. The production method for 3,3'-diallyl-4,4'-dihydroxydiphenyl sulfone according to claim 1, **characterized in that** the rearrangement reaction is carried out in the presence of an antioxidant.

7. The production method for 3,3'-diallyl-4,4'-dihydroxydiphenyl sulfone according to claim 6, **characterized in that** the rearrangement reaction is carried out in the presence of ascorbic acid as an antioxidant.

8. The production method for 3,3'-diallyl-4,4'-dihydroxydiphenyl sulfone according to claim 1, **characterized in that** the rearrangement reaction is carried out in the presence of a chelate agent.

9. The production method for 3,3'-diallyl-4,4'-dihydroxydiphenyl sulfone according to claim 8, **characterized in that** said chelate agent is ethylenediamine tetraacetic acid or a chelate agent comprising a fused ring containing a nitrogen-containing aromatic ring.

10. The production method for 3,3'-diallyl-4,4'-dihydroxydiphenyl sulfone according to claim 9, **characterized in that** the chelate agent containing a fused ring is phenanthroline.

11. A production method for 3,3'-diallyl-4,4'-dihydroxydiphenyl sulfone **characterized by** subjecting 4,4'-diallyloxydiphenyl sulfone to a rearrangement reaction under microwave irradiation at 230 to 300ºC in the presence of a basic substance or a chelate agent.
